(19) Europäisches Patentamt
European Patent Office
Office européen des brevets

(11) Veröffentlichungsnummer: **0 140 271**
**A2**

(12) # EUROPÄISCHE PATENTANMELDUNG

(21) Anmeldenummer: **84112430.8**

(22) Anmeldetag: **16.10.84**

(51) Int. Cl.[4]: **C 07 C 103/34**
C 07 C 147/12, C 07 C 149/42
C 07 D 317/58, A 01 N 37/18
A 01 N 43/30

(30) Priorität: **29.10.83 DE 3339351**

(43) Veröffentlichungstag der Anmeldung:
**08.05.85 Patentblatt 85/19**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**

(72) Erfinder: **Reinecke, Paul, Dr.**
**Lessingstrasse 11**
**D-5090 Leverkusen 3(DE)**

(72) Erfinder: **Brandes, Wilhelm, Dr.**
**Eichendorffstrasse 3**
**D-5653 Leichlingen 1(DE)**

(72) Erfinder: **Hänssler, Gerd, Dr.**
**Heymannstrasse 40**
**D-5090 Leverkusen 1(DE)**

(54) **Iodacetamide.**

(57) Iodacetamide der allgemeinen Formel (I),

$$I - CH_2 - CO - NH - R \qquad (I)$$

in welcher

R für gegebenenfalls substituiertes Cycloalkyl, für substituiertes Aralkyl oder für durch Alkylthio, Alkylsulfonyl, Halogenalkoxy, Halogenalkylthio oder Halogenalkylsulfonyl und gegebenenfalls zusätzlich durch Halogen, Cyano, Nitro, Alkyl, Halogenalkyl und Alkoxy substituiertes Aryl steht, sowie ihre Verwendung als Schädlingsbekämpfungsmittel.

Die neuen Iodacetamide können hergestellt werden, wenn man geeignete Chlor- oder Bromacetamide mit geeigneten Alkalimetalljodiden umsetzt.

EP 0 140 271 A2

0140271

BAYER AKTIENGESELLSCHAFT 5090 Leverkusen, Bayerwerk

Konzernverwaltung RP
Patentabteilung

Bas/Hed

I a

# Iodacetamide

Die Erfindung betrifft neue Iodacetamide, ein Verfahren zu ihrer Herstellung und ihre Verwendung als Schädlingsbekämpfungsmittel.

Es ist bereits bekannt geworden, daß einige Bromacetamide oder Iodacetamide, wie beispielsweise 4-Chlor-bromacetanilid, 2,4-Dichlor-bromacetanilid, Iodacetanilid oder 3,5-Dichlor-iodacetanilid fungizide Eigenschaften besitzen [vergl. z.B. J.Pharm. Soc. Japan 73, 719-721 (1953), J.Bact. 57, 339-347 (1949); J.Pharm.Soc. Japan 88, 1602-1609 (1968); US 3,642,895; Chem. Pharm.Bull.Japan 28, 2720-2733 (1980)].

Die Wirkung dieser Verbindungen ist jedoch, insbesondere bei niedrigen Aufwandmengen und -konzentrationen, nicht immer in allen Anwendungsbereichen völlig zufriedenstellend.

Es wurden neue Iodacetamide der allgemeinen Formel (I),

$$I - CH_2 - CO - NH - R \qquad (I)$$

in welcher

R für gegebenenfalls substituiertes Cycloalkyl, für substituiertes Aralkyl oder für durch Alkylthio, Alkylsulfonyl, Halogenalkoxy, Halogenalkylthio oder Halogenalkylsulfonyl und gegebenenfalls zusätzlich durch Halogen, Cyano, Nitro, Alkyl, Halogenalkyl oder Alkoxy substituiertes Aryl steht,

gefunden.

Weiterhin wurde gefunden, daß man die neuen Iodacetamide der allgemeinen Formel (I),

$$I - CH_2 - CO - NH - R \qquad (I)$$

in welcher

R für gegebenenfalls substituiertes Cycloalkyl, für substituiertes Aralkyl oder für durch Alkylthio, Alkylsulfonyl, Halogenalkoxy, Halogenalkylthio oder Halogenalkylsulfonyl und gegebenenfalls zusätzlich durch Halogen, Cyano, Nitro, Alkyl, Halogenalkyl oder Alkoxy substituiertes Aryl steht,

erhält wenn man Chlor- oder Bromacetamide der Formel (II)

$$X - CH_2 - CO - NH - R \qquad (II)$$

in welcher

X für Chlor oder Brom steht und

R die oben angegebene Bedeutung hat,

mit Alkalimetalliodiden der Formel (III),

$$A^{\oplus} I^{\ominus} \quad (III)$$

in welcher

$A^{\oplus}$ für ein Alkalimetallkation steht,

gegebenenfalls in Gegenwart eines Verdünnungsmittels umsetzt.
Schließlich wurde gefunden, daß die neuen Iodacetamide der allgemeinen Formel (I) fungizide Eigenschaften besitzen.

Ueberraschenderweise zeigen die neuen Iodacetamide der Formel (I) eine bessere fungizide Wirksamkeit als die aus dem Stand der Technik bekannten fungiziden Brom- und Iodacetanilide, wie beispielsweise das 4-Chlor-bromacetanilid, das 2,4-Dichlor-bromacetanilid, das Iodacetanilid oder das 3,5-Dichlor-iodacetanilid, welches chemisch und wirkungsmäßig naheliegende Substanzen sind. Die neuen fungiziden Iodacetamide stellen somit einen Fortschritt im Stand der Technik dar.

Die erfindungsgemäßen Iodacetamide sind durch die Formel (I) allgemein definiert. Bevorzugt sind Verbindungen der Formel (I), bei welchen

R für gegebenenfalls ein- bis fünffach, gleich oder verschieden substituiertes Cycloalkyl mit 4 bis 7 Kohlenstoffatomen oder für ein- bis dreifach, gleich oder verschieden substituiertes Aralkyl mit 6 bis 10 Kohlenstoffatomen im Arylteil und 1 oder 2 Kohlenstoffatomen im Alkylteil steht, wobei als Substituenten jeweils infrage kommen: Halogen, jeweils geradkettiges oder verzweigtes Alkyl, Alkoxy, Dioxyalkylen, Alkylthio oder Alkylsulfonyl mit jeweils 1 bis zu 4 Kohlenstoffatomen, sowie jeweils geradkettiges oder verzweigtes Halogenalkyl, Halogenalkoxy, Halogenalkylthio, Halogenalkylsulfonyl mit jeweils 1 bis 4 Kohlenstoffatomen und 1 bis 9 gleichen oder verschiedenen Halogenatomen und außerdem für ein- bis dreifach, gleich oder verschieden substituiertes Aryl mit 6 bis 10 Kohlenstoffatomen steht, wobei mindestens einer der folgenden Reste als Substituent auftritt: jeweils geradkettiges oder verzweigtes Alkylthio oder Alkylsulfonyl mit jeweils 1 bis 4 Kohlenstoffatomen oder geradkettiges oder verzweigtes Halogenalkoxy, Halogenalkylthio oder Halogenalkylsulfonyl mit jeweils 1 bis 4 Kohlenstoffatomen und 1 bis 9 gleichen oder verschiedenen Halogenatomen und wobei als zusätzliche Substituenten infrage kommen: Halogen, Cyano, Nitro, jeweils geradkettiges oder verzweigtes Alkyl oder Alkoxy mit jeweils 1 bis 4 Kohlenstoffatomen sowie geradkettiges oder verzweigtes Halogenalkyl mit 1 bis 4 Kohlenstoffatomen und 1 bis 9 gleichen oder verschiedenen Halogenatomen.

Besonders bevorzugt sind Verbindungen der Formel (I), bei welchen

R für gegebenenfalls ein- bis fünffach, gleich oder verschieden substituiertes Cyclopentyl, Cyclohexyl oder Cycloheptyl oder für ein-bis dreifach, gleich oder verschieden substituiertes Benzyl steht, wobei als Substituenten besonders infrage kommen: Fluor, Chlor, Brom, Iod, Methyl, Ethyl, Methoxy, Ethoxy, Dioxymethylen, Dioxyethylen, Methylthio, Ethylthio, Methylsulfonyl, Ethylsulfonyl, Trifluormethyl, Trichlormethyl, Dichlorfluormethyl, Difluorchlormethyl, Chlormethyl, Dichlormethyl, Trifluormethoxy, Trichlormethoxy, Trifluormethylthio, Trichlormethylthio, Trifluormethylsulfonyl oderTrichlormethylsulfonyl, außerdem für ein- bis dreifach, gleich oder verschieden substituiertes Phenyl steht, wobei mindestens einer der Substituenten Methylthio, Ethylthio, Methylsulfonyl, Ethylsulfonyl, Trifluormethoxy, Trichlormethoxy, Trifluormethylthio, Trichlormethylthio, Trifluormethylsulfonyl oder Trichlormethylsulfonyl ist, und wobei als zusätzliche Substituenten auftreten können: Fluor, Chlor, Brom, Iod, Methyl, Ethyl, Trifluormethyl, Methoxy, Ethoxy, Nitro oder Cyano.

Im einzelnen seien außer den bei den Herstellungsbeispielen genannten Verbindungen die folgenden Verbindungen der allgemeinen Formel (I) genannt:

$$I - CH_2 - CO - NH - R \quad (I)$$

| R | R |
|---|---|
| 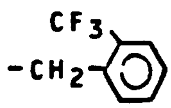 | 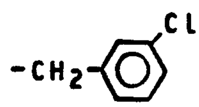 |
| 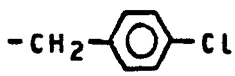 | 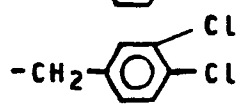 |
| 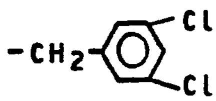 | 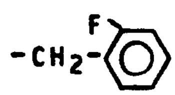 |
| 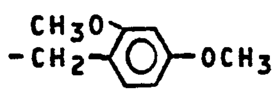 | 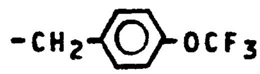 |
| 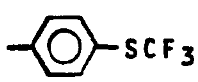 | 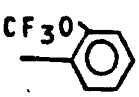 |
| 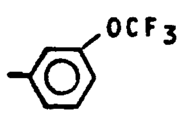 | 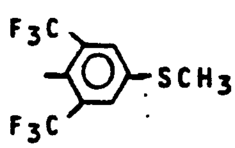 |
| 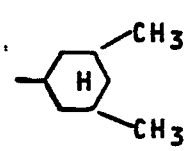 | 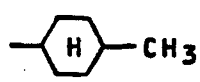 |

| R | R |
| --- | --- |
| 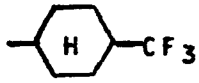 | 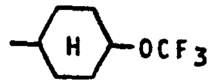 |
| 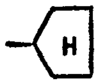 | 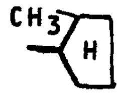 |
| 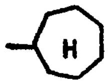 | |

Verwendet man als Ausgangsstoffe beispielsweise 2-Methylthio-chloracetamid und Natriumiodid, so läßt sich der Reaktionsablauf des erfindungsgemäßen Verfahrens durch das folgende Formelschema darstellen:

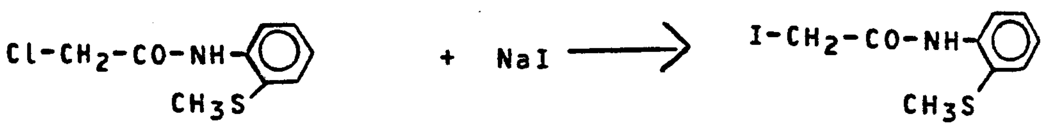

Die als Ausgangsstoffe zur Durchführung des erfindungsgemäßen Verfahrens benötigten Chlor- oder Bromacetamide sind durch die Formel (II) allgemein definiert. In dieser Formel (II) steht R für diejenigen Reste, die schon bei der Beschreibung der erfindungsgemäßen Stoffe der Formel (I) für diese Reste angegeben wurden. X steht vorzugsweise für Chlor oder Brom. Die Chlor- oder Bromacetamide der Formel (II) sind teilweise bekannt [vergl.z.B. Can.J.Chem. 44, 1247-1258 (1966)]. Die noch nicht bekannten Vertreter erhält man in

prinzipiell bekannter Weise, wenn man Chlor- oder Bromacetylhalogenide der Formel (IV),

$$X' - CO - CH_2 - X \qquad (IV)$$

in welcher
X und X' jeweils für Chlor oder Brom stehen,

mit Aminen der Formel (V)

$$R - NH_2 \qquad (V)$$

in welcher

R die oben angegebene Bedeutung hat,

gegebenenfalls in Gegenwart eines Verdünnungsmittels, wie beispielsweise Acetonitril,und gegebenenfalls in Gegenwart eines Säurebindemittels,wie beispielsweise Triethylamin,bei Temperaturen zwischen -20°C und +100°C umsetzt.
Die Chlor- bzw. Bromacetylhalogenide der Formel (IV) und die Amine der Formel (V) sind allgemein bekannte Verbindungen der organischen Chemie.

Die weiterhin zur Durchführung des erfindungsgemäßen Verfahrens als Ausgangsstoffe benötigten Alkalimetalliodide sind durch die Formel (III) allgemein definiert. In dieser Formel (III) steht $A^{\oplus}$ vorzugsweise für ein Kalium- oder Natriumkation. Die Alkalimetalliodide sind allgemein bekannte Verbindungen.

Als Verdünnungsmittel zur Durchführung des erfindungsgemäßen Verfahrens kommen inerte organische Lösungsmittel infrage. Vorzugsweise verwendet man Ketone, wie Aceton,oder Alkohole,wie Methanol oder Ethanol.

Die Reaktionstemperaturen können in dem erfindungsgemäßen Verfahren in einem größeren Bereich variiert werden. Im allgemeinen arbeitet man zwischen +20°C und +150°C, vorzugsweise zwischen +30°C und +120°C.

Zur Durchführung des erfindungsgemäßen Verfahrens setzt man pro Mol Chlor- bzw. Bromacetamid der Formel (II) im allgemeinen 1,0 bis 1,3 Mol, vorzugsweise äquimolare Mengen an Alkalimetalliodid ein. Man rührt die Reaktionsteilnehmer in dem entsprechenden Verdünnungsmittel für mehrere Stunden bei der entsprechenden Reaktionstemperatur. Zur Aufarbeitung gießt man den Reaktionsansatz in einen großen Ueberschuß Wasser und erhält das gewünschte Produkt der Formel (I) durch Absaugen und Trocknen des ungelösten Feststoffes. Die Identifizierung und Charakterisierung erfolgt mit Hilfe des Schmelzpunktes.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae,, Enterobacteriaceae, Corynebacteriaceae und Streptomycetaceae eingesetzt.
Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, zum Beispiel verursacht durch Leptosphaeria nodorum, Cochliobolus sativus, Drechslera graminea, Pyrenophora teres, Fusarium nivale und Puccinia-Arten und gegen Reiskrankheiten, wie zum Beispiel gegen den Erreger der Reisfleckenkrankheit (Pyricularia oryzae), gegen Obst- und Gemüsekrankheiten, wie zum Beispiel gegen den Erreger des Bohnengrauschimmels (Botrytis cinerea) oder gegen den Erreger des Apfelschorfes (Venturia inaequalis) oder gegen Oomyceten eingesetzt werden. Die erfindungsgemäßen Stoffe zeigen eine sehr breite Wirkung im Myzelwachstumstest und daneben auch eine gute bakterizide Wirkung.

Neben der protektiven Wirksamkeit besitzen die erfindungsgemäßen Wirkstoffe auch eine systemische Wirksamkeit und eignen sich auch als Saatgutbeizmittel.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Beispiel 1

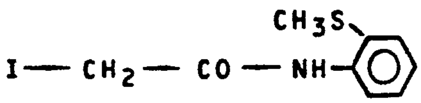

21,6g (0,1 Mol) 2-Methylthio-chloracetanilid wurden in 150 ml Alkohol gelöst und mit 16g ( ca. 0,1 Mol) Natriumiodid versetzt und 12 Stunden auf 50°C erwärmt. Nach dem Abkühlen wurde das Reaktionsgemisch auf 500ml Wasser gegossen, ausgerührt, abgesaugt und gewaschen. Nach dem Trocknen wurde aus Isopropanol umkristallisiert.
Man erhält 12,5g (41 % der Theorie) an 2-Methylthio-iodacetanilid vom Schmelzpunkt 176°C.

Herstellung der Ausgangsverbindung

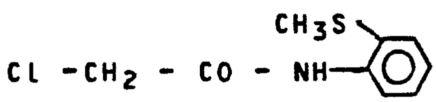

83,4g (0,3 Mol) 2-Methylthioanilin wurden in 500 ml Acetonitril gelöst und bei Raumtemperatur mit 33,9g (0,3 Mol) Chloracetylchlorid versetzt. Nach Abklingen der exothermen Reaktion wurde über Nacht bei Raumtemperatur weiter gerührt, auf etwa 1,5 l Wasser gegossen und ausgerührt. Das Reaktionsprodukt wurde abgesaugt, gewaschen und getrocknet. Es wurde aus Isopropanol umkristallisiert.
Man erhält 38,6g (60 % der Theorie) an 2-Methylthio-chloracetanilid vom Schmelzpunkt 57°C.

Beispiel 2:

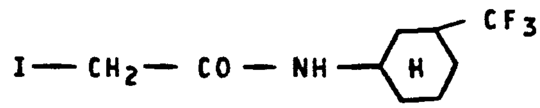

24,4g (0,1 Mol) N-(3-Trifluormethyl- cyclohexyl)-chlor-acetamid wurden in 150ml Ethanol gelöst und mit 15g (0,1 Mol) Natriumiodid versetzt. Das Reaktionsgemisch wurde 12 Stunden auf 50°C erwärmt und nach dem Abkühlen auf etwa 1 l Wasser gegossen und ausgerührt. Der ausgefallene Niederschlag wurde gewaschen und getrocknet, anschließend aus Isopropanol umkristallisiert.
Man erhält 17g (51 % der Theorie) an N-(3-Trifluormethyl-cyclohexyl)-iodacetamid vom Schmelzpunkt 132°C.

Herstellung der Ausgangsverbindung:

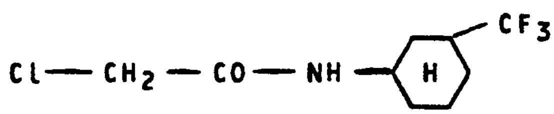

94,2g (0,3 Mol) 3-Trifluormethylcyclohexylamin wurden in 500 ml Acetonitril gelöst und bei Raumtemperatur mit 33,9g (0,3 Mol) Chloracetylchlorid versetzt. Nach Abklingen der exothermen Reaktion wurde über Nacht bei Raumtemperatur weiter gerührt und auf ca.2 l Eiswasser gegossen. Das Reaktionsprodukt wurde ausgerührt und abgesaugt, anschließend gewaschen und getrocknet. Es wurde aus Isopropanol umkristallisiert.
Man erhält 27g (74 % der Theorie) an N-(3-Trifluormethyl-cyclohexyl)-chloracetamid vom Schmelzpunkt 111°C.

In entsprechender Weise und gemäß den allgemeinen Herstellungsangaben erhält man die folgenden Verbindungen der allgemeinen Formel (I):

$$I - CH_2 - CO - NH - R \qquad (I)$$

| Bsp.-Nr. | R | Schmelzpunkt [°C] |
|---|---|---|
| 3 | 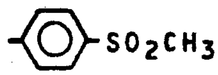 | 196 |
| 4 | 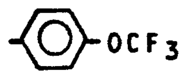 | 140 |
| 5 | 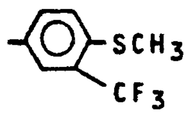 | 118 |
| 6 | 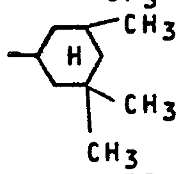 | 198 |
| 7 | 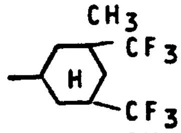 | 179 |
| 8 | 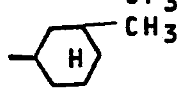 | 106 |
| 9 | 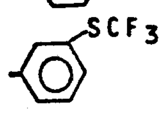 | 101 |
| 10 | 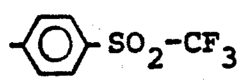 | 130 |
| 11 | 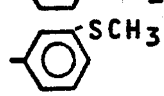 | 88 |
| 12 | 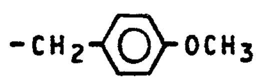 | 130 |
| 13 | 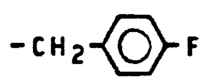 | 130 |
| 14 | 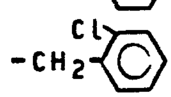 | 120 |
| 15 | 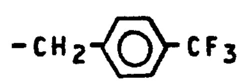 | 125 |

| Bsp.-Nr. | R | Schmelzpunkt [°C] |
|---|---|---|
| 16 | 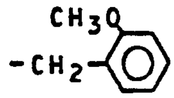 | 127 |
| 17 | 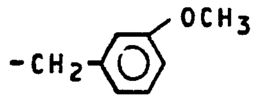 | 99 |
| 18 | 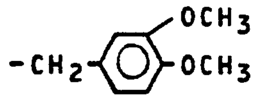 | 130 |
| 19 | 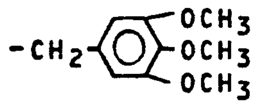 | 123 |
| 20 | 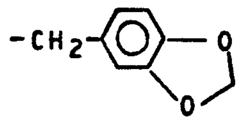 | 127 |
| 21 | 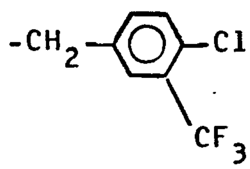 | 137 |
| 22 | 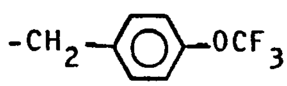 | 90 |
| 23 | 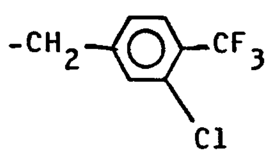 | 127 |
| 24 | 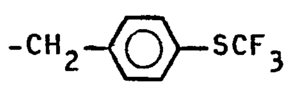 | 138 |
| 25 | 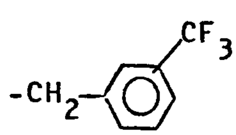 | 83 |

0140271

Anwendungsbeispiele:

In den folgenden Anwendungsbeispielen werden die nachstehend angegebenen Verbindungen als Vergleichssubstanzen eingesetzt:

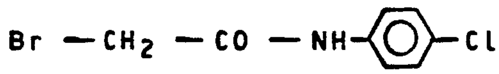 (A)

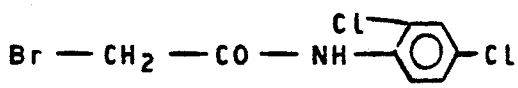 (B)

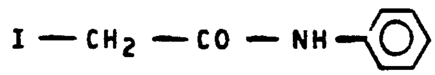 (C)

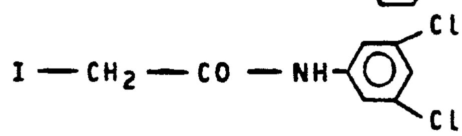 (D)

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele: 4, 7, 8, 11 und 13.

Beispiel B

Botrytis-Test ( Bohne )/protektiv

Lösungsmittel: 4,7Gewichtsteile Aceton
Emulgator: 0,3Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 10,11, 12, 13, 14, 15, 16 und 17.

Beispiel C

Drechslera graminea-Test (Gerste) / Saatgutbehandlung
(syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäß den Herstellungsbeispielen: 8 und 10.

Beispiel D

Leptosphaeria nodorum-Test (Weizen) / protektiv

Lösungsmittel: 100 Gewichtsteile Dimethylformamid
Emulgator :0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.
Die Pflanzen werden im Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß den Herstellungsbeispielen: 9, 12, 13, 14, 15 und 17.

## Patentansprüche

1. Iodacetamide der allgemeinen Formel (I)

$$I - CH_2 - CO - NH - R \qquad (I)$$

in welcher

R für gegebenenfalls substituiertes Cycloalkyl, für substituiertes Aralkyl oder für durch Alkylthio, Alkylsulfonyl, Halogenalkoxy, Halogenalkylthio oder Halogenalkylsulfonyl und gegebenenfalls zusätzlich durch Halogen, Cyano, Nitro, Alkyl, Halogenalkyl oder Alkoxy substituiertes Aryl steht.

2. Iodacetamide gemäß dem Anspruch 1, wobei in der Formel (I)

R für gegebenenfalls ein- bis fünffach, gleich oder verschieden substituiertes Cycloalkyl mit 4 bis 7 Kohlenstoffatomen oder für ein- bis dreifach, gleich oder verschieden substituiertes Aralkyl mit 6 bis 10 Kohlenstoffatomen im Arylteil und 1 oder 2 Kohlenstoffatomen im Alkylteil steht, wobei als Substituenten jeweils in Frage kommen: Halogen, jeweils geradkettiges oder verzweigtes Alkyl, Alkoxy, Dioxyalkylen, Alkylthio oder Alkylsulfonyl mit jeweils 1 bis 4 Kohlenstoffatomen, sowie jeweils geradkettiges

oder verzweigtes Halogenalkyl, Halogenalkoxy, Halogenalkylthio, Halogenalkylsulfonyl mit jeweils 1 bis 4 Kohlenstoffatomen und 1 bis 9 gleichen oder verschiedenen Halogenatomen, und außerdem für ein- bis dreifach, gleich oder verschieden substituiertes Aryl mit 6 bis 10 Kohlenstoffatomen steht, wobei mindestens einer der folgenden Reste als Substituent auftritt: jeweils geradkettiges oder verzweigtes Alkylthio oder Alkylsulfonyl mit jeweils 1 bis 4 Kohlenstoffatomen oder geradkettiges oder verzweigtes Halogenalkoxy, Halogenalkylthio oder Halogenalkylsulfonyl mit jeweils 1 bis 4 Kohlenstoffatomen und 1 bis 9 gleichen oder verschiedenen Halogenatomen und gegebenenfalls zusätzlich Halogen, Cyano, Nitro, jeweils geradkettiges oder verzweigtes Alkyl oder Alkoxy mit jeweils 1 bis 4 Kohlenstoffatomen, oder geradkettiges oder verzweigtes Halogenalkyl mit 1 bis 4 Kohlenstoffatomen und 1 bis 9 gleichen oder verschiedenen Halogenatomen.

3. Iodacetamide gemäß dem Anspruch 1, wobei in der Formel (I)

R für gegebenenfalls ein- bis fünffach, gleich oder verschieden substituiertes Cyclopentyl, Cyclohexyl oder Cycloheptyl oder für ein- bis dreifach, gleich oder verschieden substituiertes Benzyl steht, wobei als Substituenten in Frage kommen:

Fluor, Chlor, Brom, Iod, Methyl, Ethyl, Methoxy, Ethoxy, Dioxymethylen, Dioxyethylen, Methylthio, Ethylthio, Methylsulfonyl, Ethylsulfonyl, Trifluormethyl, Trichlormethyl, Dichlorfluormethyl, Difluorchlormethyl, Chlormethyl, Dichlormethyl, Trifluormethoxy, Trichlormethoxy, Trifluormethylthio, Trichlormethylthio, Trifluormethylsulfonyl oder Trichlormethylsulfonyl; für ein- bis dreifach, gleich oder verschieden substituiertes Phenyl steht, wobei mindestens einer der Substituenten Methylthio, Ethylthio, Methylsulfonyl, Ethylsulfonyl, Trifluormethoxy, Triclormethoxy, Trifluormethylthio, Trichlormethylthio, Trifluormethylsulfonyl oder Trichlormethylsulfonyl ist, und gegebenenfalls zusätzlich Fluor, Chlor, Brom, Iod, Methyl, Ethyl, Trifluormethyl, Methoxy, Ethoxy, Nitro oder Cyano.

4. Verfahren zur Herstellung von Iodacetamiden der Formel (I)

$$I - CH_2 - CO - NH - R \qquad (I)$$

in welcher

R für gegebenenfalls substituiertes Cycloalkyl, für substituiertes Aralkyl oder für durch Alkylthio, Alkylsulfonyl, Halogenalkoxy, Halogenalkylthio oder Halogenalkylsulfonyl und gegebenenfalls zusätzlich durch Halogen, Nitro,

Cyano, Alkyl, Halogenalkyl oder Alkoxy substituiertes Aryl steht, dadurch gekennzeichnet, daß man Chlor- oder Bromacetamide der Formel (II)

$$X - CH_2 - CO - NH - R \qquad (II)$$

in welcher

X für Chlor oder Brom steht und

R die oben angegebene Bedeutung hat,

mit Alkalimetalliodiden der Formel (III)

$$A^{\oplus} I^{\ominus} \qquad (III)$$

in welcher

$A^{\oplus}$ für ein Alkalimetallkation steht,

gegebenenfalls in Gegenwart eines Verdünnungsmittels umsetzt.

5. Schädlingsbekämpfungsmittel, gekennzeichnet durch einen Gehalt an mindestens einem Iodacetamid der Formel (I) gemäß den Ansprüchen 1 und 4.

6. Verfahren zur Bekämpfung von Schädlingen, dadurch gekennzeichnet, daß man Iodacetamide der Formel (I) gemäß den Ansprüchen 1 und 4 auf Schädlinge und/oder ihren Lebensraum einwirken läßt.

7. Verwendung von Iodacetamiden der Formel (I) gemäß den Ansprüchen 1 und 4 zur Bekämpfung von Schädlingen.

8. Verfahren zur Herstellung von Schädlingsbekämpfungsmitteln, dadurch gekennzeichnet, daß man Iodacetamide der Formel (I) gemäß Ansprüchen 1 und 4 mit Streckmitteln und/oder oberflächenaktiven Mitteln vermischt.